# EUROPEAN PATENT APPLICATION

(11) **EP 2 053 128 A1**
(43) Date of publication of application: **29.04.2009**
(21) Application number: 07792514.7
(22) Date of filing: 14.08.2007
(51) Int. Cl.: C12P 19/40, C12N 1/15, C12N 1/19, C12N 1/21, C12N 5/10, C12N 15/09

(54) **NOVEL METHOD FOR UTILIZATION OF MICROBIAL MUTANT**

(30) Priority: 15.08.2006 JP 2006221530
(71) Applicant: ISHIHARA SANGYO KAISHA, LTD., Osaka-shi, Osaka 550-0002 (JP)
(72) Inventor: OHSUGA, Toshio, Kusatsu-shi, Shiga 525-0025 (JP); FUSHIKIDA, Kuni, Kusatsu-shi, Shiga 525-0025 (JP)
(74) Representative: Hartz, Nikolai
(86) International application number: PCT/JP2007/065871
(87) International publication number: WO 2008/020595

(57) **Abstract**

The object of the present invention is to provide a microbial mutant capable of producing S-adenosylmethionine (hereinafter referred to as SAM) in a large amount with no change in growth characteristics and a method of efficiently producing SAM by incubating the microbial mutant.

The method of producing S-adenosylmethionine comprises incubating a microorganism having a loss or a reduction in the function of an enzyme relating to synthesis of phosphatidylcholine and recovering S-adenosylmethionine accumulated in the culture.

## Description

### TECHNICAL FIELD

The present invention relates to a microbial mutant capable of producing S-adenosylmethionine (hereinafter referred to as SAM) in a large amount and use thereof.

### BACKGROUND ART

SAM is a biologically active substance present in all the body tissues which functions as a methyl donor in various methylation reactions in synthesis and metabolism of hormones, neurotransmitters, phospholipids and proteins. Because it clinically enhances liver function and helps elimination of harmful substances from the body, it has been known to therapeutically effective against liver disease, hyperlipidemia, arteriosclerosis and the like. It is also associated in synthesis of neurotransmitters and is used as a therapeutic agent and a supplement for depression, Alzheimer's disease and arthritis in recent years.

Currently, SAM is industrially produced by fermentation of microorganisms, mainly yeasts. As the yeast strains used for its production, existing ones known to accumulate SAM to a high concentration by past experience and research are used, and its production has been increased through studies of culture conditions such as culture medium compositions.

Because the demand for SAM is increasing as the usefulness of SAM becomes apparent, more productive SAM accumulating strains are demanded. However, with no efficient way to select SAM accumulating strains, it is quite difficult to obtain more productive mutant strains from existing SAM producing strains. In addition, the conventional selective breeding has its limitations and cannot provide strains capable of accumulating SAM to a drastically high concentration.

Meanwhile, the recent advances in biotechnology have made available well-organized data on metabolic pathways and genes in microorganisms and have enabled putative identification of genes associated with the metabolism and accumulation of SAM and direct manipulation of particular genes, and have provided a new approach for production of mutant strains.

Examples of SAM accumulating mutant strains produced through the above-mentioned approach include a yeast mutant with a mutation in the CYS4 gene encoding cystathionine β synthase, which is an enzyme participating in part of the SAM metabolic pathway (patent document 1), and a yeast mutant with a mutation in the SAH1 gene encoding S-adenosylhomocysteine hydrolase (patent document 2). However, the low growth rates resulting from the mutations are problematic. In general, not only SAH1 mutants but also many other mutants show altered growth characteristics resulting from mutations and take longer to grow or require special nutrients.
Patent document 1: JP-A-2001-112474
Patent document 2: JP-A-2005-261361

### DISCLOSURE OF THE INVENTION

The object of the present invention is to provide a microbial mutant capable of producing SAM in a large amount with no change in growth characteristics and use thereof.

### MEANS OF SOLVING THE PROBLEMS

As a result of their extensive research on various SAM-related enzymes, the present inventors found that a mutant capable of accumulating SAM to a high concentration intracellularly or extracellularly can be obtained by molecular biologically suppressing the function of a SAM-consuming enzyme relating to biosynthesis of phosphatidylcholine and have accomplished the present invention on the basis of the discovery.

Namely, the present invention is summarized as follows:
(1) A method of producing S-adenosylmethionine, which comprises incubating a microorganism having a loss or reduction in the function of an enzyme relating to synthesis of phosphatidylcholine and recovering S-adenosylmethionine accumulated in the culture.
(2) The method according to (1), wherein the enzyme relating to synthesis of phosphatidylcholine is phosphatidylethanolamine methyltransferase and/or N-methylphosphatidylethanolamine methyltransferase.
(3) The method according to (1), wherein the enzyme relating to synthesis of phosphatidylcholine is phosphatidylethanolamine methyltransferase.
(4) The method according to (1), wherein the microorganism is a yeast.
(5) The method according to (4), wherein the yeast is Saccharomyces cerevisiae.
(6) The method according to (1), wherein the microorganism is a mutant having a substitution, deletion, insertion and/or addition of one or more nucleotides in the sequence of a gene encoding an enzyme relating to synthesis of phosphatidylcholine.
(7) The method according to (6), wherein the enzyme relating to synthesis of phosphatidylcholine is phosphatidylethanolamine methyltransferase.
(8) The method according to (6), wherein the mutant further has substitution, deletion, insertion and/or addition of one or more nucleotides in the sequence of a gene encoding cystathionine β synthase and/or an enzyme relating to biosynthesis of ergosterol.
(9) A microbial mutant having a substitution, deletion, insertion and/or addition of one or more nucleotides in the sequence of a gene encoding an enzyme relating to synthesis of phosphatidylcholine and having a loss or reduction of the enzymatic function.
(10) The microbial mutant according to (9), wherein the enzyme relating to synthesis of phosphatidylcholine is phosphatidylethanolamine methyltransferase.
(11) The microbial mutant according to (10), which further has a substitution, deletion, insertion and/or addition of one or more nucleotides in the sequence of a gene encoding cystathionine β synthase and/or an enzyme relating to biosynthesis of ergosterol and having a loss or reduction of the enzymatic function.

### EFFECTS OF THE INVENTION

The microbial mutant of the present invention accumulates SAM to a high concentration intracellularly or extracellularly and makes it possible to produce SAM more efficiently in a large amount than before. The microbial mutant of the present invention does not differ in growth characteristics from the parent strain and enables efficient production without modifying the conventional incubation conditions.

### BRIEF DESCRIPTION OF THE DRAWINGS

[Fig. 1] The relation between the SAM-related metabolic pathway and genes in Saccharomyces cerevisiae.
[Fig. 2] Agarose gel electrophoresis showing an insertion of a vector sequence into the target genomic site.
[Fig. 3] The SAM content per unit amount of medium in cultures of the parent strain (Wt) and the CH02 mutant.
[Fig. 4] TLC plate analysis of the total phospholipids extracted from 24-hour cultures of the parent strain (Wt) and CHO2 mutant.
[Fig. 5] The growth curve of the parent strain (Wt) and CH02 mutant in YPD and SAM fermentation medium over 72 hours.
[Fig. 6] Agarose gel electrophoresis showing insertion of a vector sequence into a target genomic site and retention of the mutation in the CH02 gene.
[Fig. 7] The SAM content per unit amount of medium in cultures of the parent strain (Wt) and the CH02 CYS4 double mutant.

### BEST MODE FOR CARRYING OUT THE INVENTION

Now, one mode for carrying out the present invention will be described, but the present invention is by no means restricted thereto.

The microbial mutant of the present invention is a microorganism capable of accumulating SAM intracellularly or extracellularly and a microbial mutant having a mutation in a gene relating to the function of an enzyme relating to biosynthesis of phosphatidylcholine.

Phosphatidylcholine is one of the phospholipids constituting cell membranes and an important constituent of the body universally found in eukaryotes. Its biosynthetic pathway is universally conserved among all eukaryotes, and it is known that yeasts used for production of SAM have similar synthetic pathways.

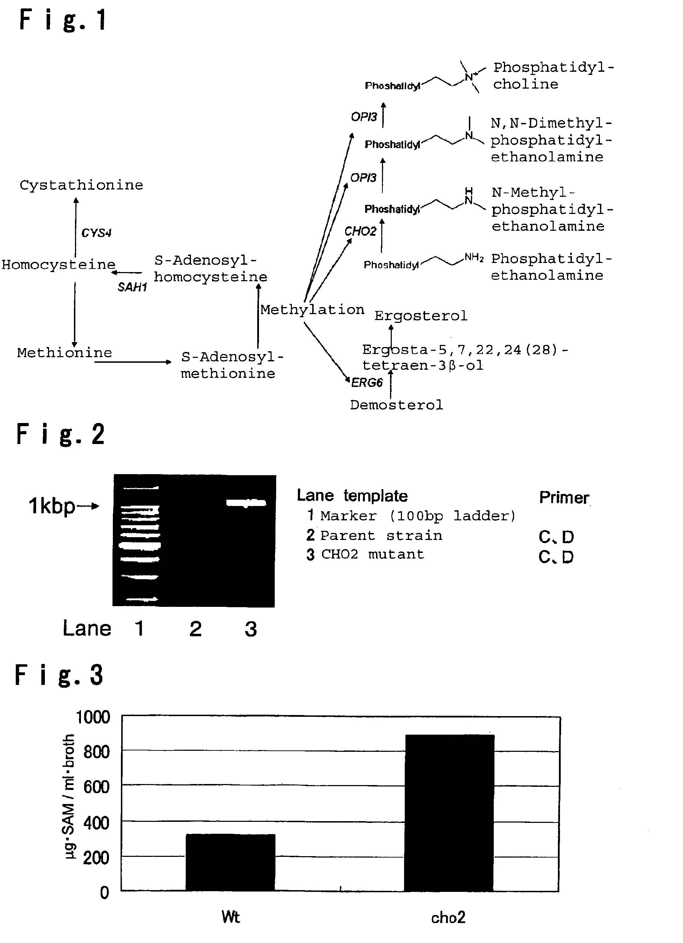

Among the enzymes relating to its biosynthetic pathway are enzymes relating to biosynthesis of phosphatidylcholine. These enzymes catalyze a series of reactions for synthesis of phosphatidylcholine from phophatidylethanolamine and specifically mean phosphatidylethanolamine methyltransferase (such as CH02), which catalyzes the methylation of the amino group in phosphatidylethanolamine, and N-methylphosphatidylethanolamine methyltransferase (such as OP13), which catalyzes the subsequent N-methylation of N-methylphosphatidylethanolamine to phosphatidylcholine. It is known that SAM participates in the methylation reactions as the methyl donor (Fig. 1).

On the hypothesis that in the above-mentioned biosynthetic pathway, the loss of the enzymatic function of an enzyme relating to biosynthesis of phosphatidylcholine by mutation of the gene encoding it reduces the SAM consumption in the biosynthesis of phosphatidylcholine from phosphatidylethanolamine and hence allows intracellular and extracellular accumulation of SAM, a gene encoding an enzyme relating to biosynthesis of phosphatidylcholine in yeast was mutated, as a result, the resulting mutant was found to be capable of accumulating SAM to a high concentration. Namely, the microbial mutant of the present invention with mutation in a gene relating to an enzyme relating to biosynthesis of phosphatidylcholine can accumulate SAM to a high concentration.

The gene encoding an enzyme relating to biosynthesis of phosphatidylcholine may be a gene encoding phosphatidylethanolamine methyltransferase or N-methylphosphatidylethanolamine methyltransferase, and for example, the phosphatidylethanolamine methyltransferase gene (Saccharomyces genome database, registry number: YGR157W) and the N-methylphosphatidylethanolamine methyltransferase gene (YJR073C) in Saccharomyces cerevisiae may be mentioned.

Herein, "microbial mutant" means a strain with a loss or a reduction in the function of the above-mentioned enzyme, as compared with the wild-type strain or its parent strain. Specifically speaking, it is a microorganism which can no longer produce the enzyme protein in the normal form due to a substitution, deletion, insertion and/or addition of one or more nucleotides in the sequence of the gene encoding the enzyme, or, alternatively, a microorganism with significantly lower expression of the gene encoding the enzyme. The mutant of the present invention includes, for example, a microorganism in which the function of the enzyme is considerably suppressed as a result of disruption of an expression regulatory domain, introduction of a repression domain or post-transcriptional expression regulation (such as RNAi).

Further, the microbial mutant of the present invention may be a multiple mutant in which an enzyme relating to biosynthesis of phosphatidylcholine and other SAM-related enzymes such as cystathionine β synthase, S-adenosylhomocysteinase and enzymes relating to biosynthesis of ergosterol have been mutated to increase the ability to accumulate SAM.

Cystathionine β synthase is an enzyme (such as CYS4) which catalyzes synthesis of cystathionine from homocysteine and serine, while S-adenosylhomocysteinase is an enzyme (such as SAH1) which catalyzes synthesis of homocysteine from S-adenosylhomocysteine. As shown in Fig. 1, these enzymes (CYS4 and SAH1) participate in SAM metabolism, and mutants with mutation in the genes encoding these enzymes are known to accumulate SAM (patent documents 1 and 2).

The enzymes relating to biosynthesis of ergosterol mean a series of enzymes (such as ERG6) relating to the ergosterol biosynthetic pathway. Mutants having a deficiency in the ergosterol biosynthetic pathway are known to accumulate SAM. SAM serves as the methyl donor in the synthesis of ergosta-5,7,22,24(28)-tetraen-3β-ol from demosterol in the biosynthetic pathway (Fig. 1).

Herein, "a multiple mutant" means a strain with a loss or a reduction in the functions of more than one enzyme in a single cell. However, complete blocking of a metabolic pathway associated with an important biologically active substance like SAM usually has influence on the cell growth. For example, it is known that multiple mutants of laboratory yeast with mutations in SAM1 and SAM2 encoding SAM synthetases are nonviable. Thus, it is important to avoid complete blocking of a particular metabolic pathway in order to minimize the influence of the growth (growth characteristics), and it is preferred to combine mutations relating to different metabolic pathways. Therefore, in order to obtain a multiple mutant, it is preferred to combine a mutation relating to phosphatidylcholine biosynthesis, with a mutation in cystathionine β synthase involved in the SAM metabolic pathway or a mutation relating to a different metabolic pathway such as the ergosterol biosynthetic pathway, and thereby it is possible to minimize influence on the growth, i.e., the change in growth characteristics.

The desired mutant can be obtained by any methods without any particular restrictions, for example, by a method utilizing homologous recombination, which allows transformation by sequence targeted mutagenesis using a DNA sequence homologous to a target gene designed to cause various mutations which leads to a loss of an enzymatic function after homologous recombination. Mutations may be introduced by other ordinary methods, for example, physically by UV irradiation or radioactive ray (such as γ ray) irradiation, or chemically by suspending cells in mutagens such as nitrous acid, nitrogen mustard, acridine dyes, ethyl methanesulfonate (EMS), N-methyl-N'-nitro-N-nitrosoguanidine (NTG), or by appropriate combinations thereof.

The desired mutants may be selected by any methods without particular restrictions, and can be selected efficiently if a selective marker is incorporated in the vector used for the above-mentioned homologous recombination. Known selectable markers such as dye-producing genes, drug resistance genes, lethal genes or the like are available, and for example, clones carrying vectors can be selected under appropriate selective conditions. In addition, direct gene analyses, specifically speaking, analyses of changes in the sequence of the gene encoding the enzyme due to mutation, deletion and/or insertion by PCR or sequencing may be mentioned. Alternatively, analyses of specific characteristics may be mentioned, analyses of the mRNA of the gene or expression of the protein from the gene, assays of the functional activity of the enzyme, analyses of change in the amount of the metabolite resulting from the catalysis by the enzyme can be used, and these methods can be used in combination.

The species used as the parent strain may be any living organism capable of biosynthesis of phosphatidylcholine and is preferably a microorganism because it is easy to grow. Specific examples include yeasts, filamentous fungi, Basidiomycetes and the like, and among them, use of a yeast is preferred because of its high ability to accumulate SAM known from past studies.

Yeasts are unicellular fungi and include ascosporogenous yeasts, basidiomycetous yeasts, imperfect yeasts and the like. Among them, ascosporogenous yeasts such as Saccharomyces, Picha, Hansenula and Zygosaccharomyces are preferred, and in particular Saccharomyces cerevisiae is are preferred. Specific examples include laboratory yeast, sake yeasts, shochu yeasts, wine yeasts, brewer's yeasts and baker's yeasts.

SAM can be produced in high yields by incubating the microbial mutant of the present invention in an appropriate medium to allow the mutant to produce SAM and recovering the SAM intracellularly or extracellularly accumulated by an appropriate technique.

There are no particular restrictions on how to incubate the microbial mutant of the present invention. The incubation conditions suitable for the parent strain may be used. For example, it can be incubated aerobically at an incubation temperature of 25°C to 45°C at a pH adjusted to 5-8 for 16 to 120 hours. For the pH adjustment, acidic or alkaline compound that is inorganic or organic, ammonia gas and the like may be used.

Although there are no particular restrictions on the culture medium, a culture medium suitable for the parent strain is preferably used. The culture medium preferably contains a carbon source, a nitrogen source, and inorganic ions necessary for microorganisms or microbial mutants to grow. As the carbon source, a monosaccharide such as glucose or fructose, a disaccharide such as sucrose or lactose, a polysaccharide such as cellulose or starch, an organic compound such as ethanol or lactic acid, a crude material such as black treacle or the like may be used. As the nitrogen source, for example, an inorganic salt such as an ammonium salt or a nitrate, a nitrogen-containing organic compound such as an amino acid or glucosamine, an organic material such as yeast extract or peptone, or the like may be used. In addition to these basic components, inorganic ion salts, vitamins, minerals, organic compounds, buffering agents, antifoaming agents and the like suitable in terms of fermentation engineering may be incorporated. It is particularly preferred to use a methionine-supplemented culture medium for still higher SAM yields. Methionine is added in an amount of at least 0.01%, preferably at least 0.05%, particularly preferably from 0.1 to 0.3%, based on the culture medium. Herein, the percentages (%) representing the amounts and contents of components in the medium are W/V(%).

There are no particular restrictions on how to extract and isolate SAM from the culture after incubation. Cells can be collected from the culture by centrifugation, sedimentation or filtration. For example, yeast cells can easily be collected by centrifugation followed by filtration. SAM can be recovered from the cells by physical disruption (with a homogenizer, with glass beads or by freezing and thawing) or by chemical disruption (by solvent treatment, acid or base treatment, osmotic treatment or enzymatic treatment). For example, SAM is readily extracted and recovered from the yeast by acid treatment. Further, the SAM extract can be purified by conventional purification methods (such as solvent extraction, column chromatography and salt precipitation). For example, SAM can be purified by acidic ion exchange chromatography as well as by salt precipitation by addition of acetone or the like. These methods may be combined appropriately, if necessary.

Now, the present invention will be described by reference to specific examples. However, the present invention is by no means restricted to these examples.

### EXAMPLE 1 SAM PRODUCTION BY CH02 MUTANT

A laboratory yeast (Saccharomyces cerevisiae BY20592) was used as the parent strain, and the disruption of its phosphatidylethanolamine methyltransferase gene (CH02) was carried out. All the yeast strains used herein were obtained from the Yeast Genetic Resource Center.

### (1) Preparation of gene disruption vector

A vector for disruption of the target gene was prepared from pAUR135 vector, TAKARA BIO. The EcoRI-SmaI restriction fragment of pAUR135 vector and the EcoRI restriction fragment of a PCR amplification product from CH02 (sequence amplified by PCR using primers A and B (Table 1, SEQ ID NOS: 1 and 2) and the genomic DNA of the laboratory yeast BY2041 as the template) were mixed and ligated with T4DNA ligase to give a CH02 disruption vector. The vector was cloned into Escherichia coli DH5α, and the plasmid was isolated from the E. coli culture by an ordinary method to obtain a necessary amount of the gene disruption vector.

### (2) Disruption of yeast gene

The above-mentioned vector was used for yeast gene disruption. The laboratory yeast (Saccharomyces cerevisiae BY20592) as the parent strain was transformed with the above vector by the lithium acetate method. The transformed yeast was incubated overnight in YPD liquid medium and plated on a YPD solid medium supplemented with 0.5 µg/ml aureobasidin A, and viable colonies were obtained.

Insertion of the vector sequence in the target genomic sequence was confirmed by PCR using a portion of the colonies as the template. PCR was carried out using primers which anneal to the genomic sequence around the vector insertion site and a sequence from the pAUR135 vector, and the presence of the amplification product of the target sequence (1 kbp) was checked by agarose gel electrophoresis followed by ethidium bromide staining. For the confirmation of the CH02 mutant, primers C and D (Table 1, SEQ ID NOS: 3 and 4) were used. As a result, the CH02 mutant gave a band of about 1 kbp, which indicates insertion of the vector sequence at the right site for disruption of the target gene (Fig. 2).

### (3) SAM production by mutants

The CH02 mutant obtained in (2) was grown under the following conditions, and a culture of the mutant was obtained. The parent strain and the mutant were separately inoculated into 5 ml of SAM fermentation medium (5% glucose, 1% peptone, 0.5% yeast extract, 0.4% KH2PO4, 0.2% K2HP04, 0.05% Mg2SO4·H2O, 0.15% L-methionine, pH 6.0) in 50 ml centrifuge tubes and incubated with shaking at 28°C for 72 hours to obtain sufficient amounts of cultures.

SAM accumulated in the cultures was extracted as follows and assayed. The cells were precipitated by centrifugation, and the supernatants were removed. 10% perchloric acid was added to extract SAM (room temperature, 1 hour). The supernatants were separated by paper chromatography (developing solvent EtOH : n-BuOH : water :AcOH : 1% NaP2O7 = 30:35:40:1:2, and the SAM spots was cut out and extracted to obtain assay samples.

For the assay, HPLC was performed using the UV absorption at 260 nm in relation to that of a standard sample as an index. The analytical conditions were as follows: instrument: Waters 2690 Separation Module - Waters 2487 Dual Absorbance Detector system, column: Cosmosil packed column 5C18-MS (4.6 i.d. x 250 mm), elution solvent: 5% methanol-95% 0.2 M KH2PO4, flow rate: 1 ml/min, column temperature: 25°C.

As a result, it turned out that the CH02 mutant accumulated 2.8 times SAM than the parent strain in terms of the SAM content per unit amount of medium (Fig. 3).

### EXAMPLE 2 CONFIRMATION OF LOSS OR REDUCTION IN ENZYMATIC FUNCTION

### (1) Confirmation of loss of the enzymatic function from gene mutation

The phospholipid composition, especially the phosphatidylcholine content, in the CH02 mutant obtained in Example 1 was measured to confirm a loss or reduction of the enzymatic function and was compared with that in the parent strain.

Each mutant was incubated in YPD medium (2% peptone, 1% yeast extract, 2% glucose) at 28°C for 24 hours with shaking, and the total phospholipids were extracted from the cells, and the phospholipid composition was analyzed by silica gel thin layer chromatography (TLC).

The cells were collected by centrifugation, then extracted with the same amount of an organic solvent {CHCl3-MeOH (2:1)} as the culture medium over 1 hour and washed twice with one-fifth as much water as the organic solvent. The organic layer was recovered and evaporated to dryness. The residue was dissolved in chloroform again to obtain an analytical sample.

The sample was spotted on a silica gel TLC plate and separated by TLC (developing solvent: CHCl3-MeOH-AcOH (20:10:3)), and the plate was immersed 5% phosphomolybdic acid-ethanol and heated, and the loss of the enzyme function was confirmed from the change in the phospholipids composition represented by the resulting spots of organic compounds.

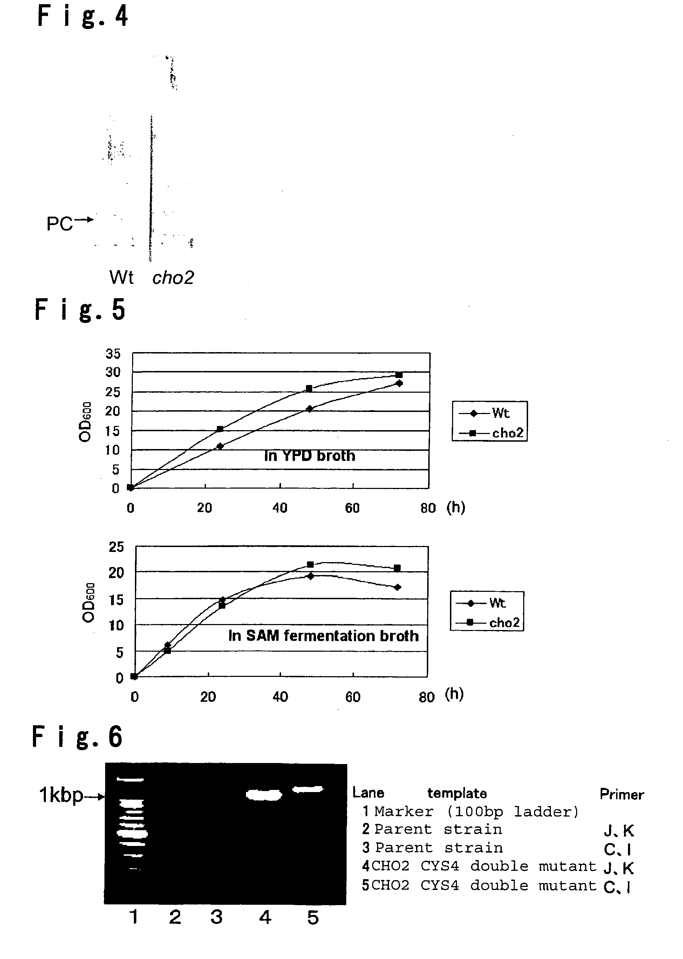

The result was that there were a reduction in the phosphatidylcholine in the total phospholipids and a change in the lipid composition resulting from the mutation, which indicate a deficiency in the biosynthetic pathway from phosphatidylethanolamine to phosphatidylcholine (Fig. 4). However, phosphatidylcholine did not completely disappear, though decreased, in the mutant, which can be explained from the fact that another phosphatidylcholine biosynthetic pathway is known to exist in addition to the pathway catalyzed by CH02 which was blocked in the mutant. The presence of residual phosphatidylcholine in the mutant which is essential for homeostasis in microorganisms is advantageous for normal growth of the mutant (growth characteristics).

### (2) Confirmation of growth characteristics

In order to confirm whether the mutation changed the growth characteristics, 5 mL of culture media (SAM fermentation medium and YPD medium) in 50 mL centrifuge tubes were inoculated and incubated with shaking at 28°C for 48 hours, and the growth characteristics were evaluated from the OD600 values. The results indicate that the CH02 mutant retained the same growth characteristics as the parent strain (Fig. 5).

### EXAMPLE 3 IMPROVED SAM PRODUCTIVITY IN CH02 CYS4 DOUBLE MUTANT

The CH02 mutant has such excellent characteristics that it accumulates SAM to a high concentration while retaining the growth characteristics inherited from its parent. To develop a mutant capable of accumulating more SAM than the CH02 mutant, disruption of the cystathionine β synthase, gene (CYS4) in the CH02 mutant was attempted.

The CH02 disruption vector prepared in Example 1 was amplified in full-length along part of the CH02 gene in the CH02 disruption vector by using primers E and F (Table 1, SEQ ID NOS: 5 and 6) with a point mutation introduced so as to generate a stop codon at a sequence corresponding to CDS. The PCR product was directly used for transformation of Escherichia coli DH5α, and an Escherichia coli strain carrying the CH02 disruption vector with a point mutation as desired was obtained. The plasmid was isolated from the Escherichia coli culture to obtain a necessary amount of a CH02 disruption vector with a point mutation (hereinafter referred to as CH02 mutated vector).

The CH02 mutated vector was used to mutate the CH02 gene in the yeast genome. The vector was transformed into a laboratory yeast (Saccharomyces cerevisiae BY20592) as the parent strain by the lithium acetate method. The transformed yeast was incubated in YPD liquid medium overnight and plated on YPD solid medium supplemented with 0.5 µg/ml aureobasidin A, and the viable colonies were checked for insertion of the vector at CH02 site by PCR.

The colonies were plated on YP-Galactose solid medium (2% peptone, 1% yeast extract, 2% galactose, 2% purified agar) and incubated at 28°C for 3 days. Because of the galactose-inducible lethality of the pAUR135 vector, viable colonies are vector-removed reverse mutants, some of which are strains carrying the stop codon from the CH02 mutated vector in the genome. Colonies carrying a stop codon in CH02 (hereinafter referred to vector-removed CH02 mutants) were selected by genome sequencing. These strains without the vector sequence are retransformable and were used for the subsequent experiment.

Then, a CYS4 disruption vector was prepared. Specifically speaking, the KpnI-SmaI restriction fragment of pAUR135 vector and the KpnI restriction fragment of a PCR amplification product from CYS4 (sequence amplified by PCR using primers G and H, (Table 1, SEQ ID NOS: 7 and 8) and the genomic DNA of the laboratory yeast BY2041 as the template) were mixed and ligated with T4DNA ligase to give a CYS4 disruption vector. The vector was cloned into Escherichia coli DH5α, and the plasmid was isolated from the Escherichia coli culture by an ordinary method to obtain a necessary amount of the gene disruption vector.

The above-mentioned vector was used for disruption of each gene. The vector-removed CH02 mutant as the parent strain was transformed with each of the vector by the lithium acetate method. The transformed yeast was incubated overnight in YPD liquid medium and plated on a YPD solid medium supplemented with 0.5 µg/ml aureobasidin A, and the viable colonies were checked for insertion of the vector at the target genomic sequence by PCR using primers which anneal to the genomic sequence around the vector insertion site and a sequence from the pAUR135 vector. In order to confirm retention of the mutation in the CH02 gene, PCR was carried out using primers specific to the mutated site, and the amplification product of the target sequence (about 1 kbp) was checked by agarose electrophoresis followed by ethidium bromide staining. For the confirmation of insertion of the vector in the CYS4 gene, primers C and I (Table 1, SEQ ID NOS: 3 and 9) were used, and for the confirmation of the CH02 mutation, primers J and K (Table 1, SEQ ID NOS: 10 and 11) were used. As a result, the mutant gave a band of about 1 kbp, which indicates insertion of the vector sequence at the right site for disruption of the target gene and retention of the mutation in the CH02 gene (Fig. 6). The mutant is referred to as CH02 CYS4 double mutant.

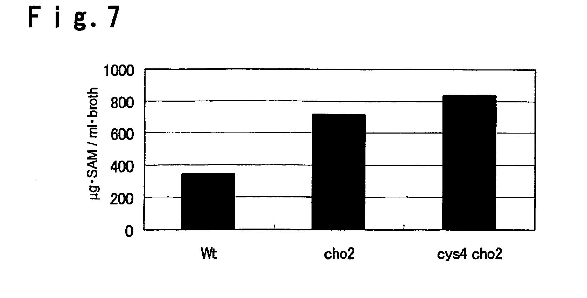

The SAM content in the CH02 CYS4 double mutant and the growth characteristics were determined as by previously described, and as a result, the SAM yield from the mutant was 1.2 times higher than that from the CH02 mutant (Fig. 7), which indicates improvement in SAM productivity by transformation into a CH02 CYS4 double mutant.

The sequences of the primers used in the Examples are shown in Table 1.

**TABLE 1**

| Primer ID | Nucleotide sequence | SEQ ID NO: |
|---|---|---|
| Primer A | CACGATATGGTGCGTTCGC | 1 |
| Primer B | GGAATTCCGCTACGGTACCAATTGTG | 2 |
| Primer C | GTAAAACGACGGCCAGT | 3 |
| Primer D | TCGAAGCCAGAACACGTTCG | 4 |
| Primer E | CGTTTACCTTTCTATTTGATAAAGCTGGGTTC | 5 |
| Primer F | GAACCCAGCTTTATCAAATAGAAAGGTAAACG | 6 |
| Primer G | AACCCGGGACTTAATCTGAGTAGCAAGCCGATTCAAGAC | 7 |
| Primer H | GGGGTACCGCGTTACCAAACACATCAGCG | 8 |
| Primer I | GGACATCTAGATAAATACGACG | 9 |
| Primer J | ATTTGAACCCAGCTTTATCA | 10 |
| Primer K | CAGAAACGGCAAAACCTC | 11 |

### SEQUENCE LISTING FREE TEXT

SEQ ID NO: 1, Primer for amplification of CH02 gene
SEQ ID NO: 2, Primer for amplification of CH02 gene
SEQ ID NO: 3, Primer for amplification of pAUR135 vector
SEQ ID NO: 4, Primer for amplification of CH02 gene
SEQ ID NO: 5, Mutagenic primer containing mutations for amplification of CH02 gene
SEQ ID NO: 6, Mutagenic primer containing mutations for amplification of CH02 gene
SEQ ID NO: 7, Primer for amplification of CYS4 gene
SEQ ID NO: 8, Primer for amplification of CYS4 gene
SEQ ID NO: 9, Primer for amplification of CYS4 gene
SEQ ID NO: 10, Primer for amplification of CH02 gene mutations
SEQ ID NO: 11, Primer for amplification of CH02 gene

### INDUSTRIAL APPLICABILITY

The microbial mutant of the present invention is capable of accumulating S-adenosymethionine intracellularly, and use of the microbial mutant of the present invention enables mass production of S-adenosylmethionine, which is useful as a therapeutic agent for depression and the like or a supplement. Therefore, the present invention is applicable in the pharmaceutical industry.

The entire disclosure of Japanese Patent Application No. 2006-221530 filed on August 15, 2006 including specification, claims, drawings and summary is incorporated herein by reference in its entirety.

## Claims

1. A method of producing S-adenosylmethionine, which comprises incubating a microorganism having a loss or reduction in the function of an enzyme relating to synthesis of phosphatidylcholine and recovering S-adenosylmethionine accumulated in the culture.

2. The method according to Claim 1, wherein the enzyme relating to synthesis of phosphatidylcholine is phosphatidylethanolamine methyltransferase and/or N-methylphosphatidylethanolamine methyltransferase.

3. The method according to Claim 1, wherein the enzyme relating to synthesis of phosphatidylcholine is phosphatidylethanolamine methyltransferase.

4. The method according to Claim 1, wherein the microorganism is a yeast.

5. The method according to Claim 4, wherein the yeast is Saccharomyces cerevisiae.

6. The method according to Claim 1, wherein the microorganism is a mutant having substitution, deletion, insertion and/or addition of one or more nucleotides in the sequence of a gene encoding the enzyme relating to synthesis of phosphatidylcholine.

7. The method according to Claim 6, wherein the enzyme relating to synthesis of phosphatidylcholine is phosphatidylethanolamine methyltransferase.

8. The method according to Claim 6, wherein the mutant further has a substitution, deletion, insertion and/or addition of one or more nucleotides in the sequence of a gene encoding cystathionine β synthase and/or an enzyme relating to biosynthesis of ergosterol.

9. A microbial mutant having a substitution, deletion, insertion and/or addition of one or more nucleotides in the sequence of a gene encoding an enzyme relating to synthesis of phosphatidylcholine and having a loss or reduction of the enzymatic function.

10. The microbial mutant according to Claim 9, wherein the enzyme relating to synthesis of phosphatidylcholine is phosphatidylethanolamine methyltransferase.

11. The microbial mutant according to Claim 10, which further has a substitution, deletion, insertion and/or addition of one or more nucleotides in the sequence of a gene encoding cystathionine β synthase and/or an enzyme relating to biosynthesis of ergosterol and having a loss or reduction of the enzymatic function.
